# EUROPEAN PATENT APPLICATION

(11) **EP 2 407 161 A1**
(43) Date of publication of application: **18.01.2012**
(21) Application number: 10305779.0
(22) Date of filing: 13.07.2010
(51) Int. Cl.: A61K 31/09, A61K 39/395, A61P 35/00

(54) **An antitumoral combination comprising ombrabulin and bevacizumab**

(71) Applicant: SANOFI, 75013 Paris (FR)
(72) Inventor: The designation of the inventor has not yet been filed
(74) Representative: Gaslonde, Aude

(57) **Abstract**

The invention concerns an antitumoral combination comprising ombrabulin and bevacizumab, and its use in the treatment of advanced solid tumors.

## Description

The invention relates to an anti-tumoral combination comprising ombrabulin and bevacizumab and its use in the treatment of advanced solid tumors.

### [Prior art and problem to be solved]

WO 99/51246 discloses the ombrabulin/platinum salt combination.

WO 2004/037258 discloses the combination of ombrabulin with various anti-tumoral agents chosen among taxanes (paclitaxel, docetaxel), alkylant agents (cyclophosphamide, isofosfamide,...), antimetabolites (5-FU, cytarabine,...), epidophylloptoxine, antibiotics (doxorubicine,...), vinca alcaloids.

EP 1407784 discloses ombrabulin / dexamethasone combination.

WO 2007/077309 discloses ombrabulin/aflibercept combination.

There is still a need to find and optimize new therapeutic options, including synergic combinations, to treat patients with advanced solid tumors.

The invention meets this need by providing a new pharmaceutical anti-tumoral combination comprising ombrabulin and bevacizumab for which doses of each component and a suitable administration protocol have been determined, in order to obtain a well tolerated combination therapy which does not exacerbate the toxicity of each of the anti-tumoral agents and which allows the treatment of advanced solid tumors either by stabilizing or by leading to a partial or complete regression of the tumor.

### [Description of the invention]

The invention is an anti-tumoral combination of ombrabulin and bevacizumab; ombrabulin being in the form of a free base or of an addiction salt with a pharmaceutical acceptable acid, or in the form of a hydrate or of a solvate.
The anti-tumoral combination of the invention is well tolerated, does not exacerbate the toxicity of each of the anti-tumoral agents and allows the treatment of advanced solid tumors either by stabilizing or by inducing a partial or complete regression of the tumor.

Ombrabulin (AVE8062) belongs to the family of combretastatins and has the formula: 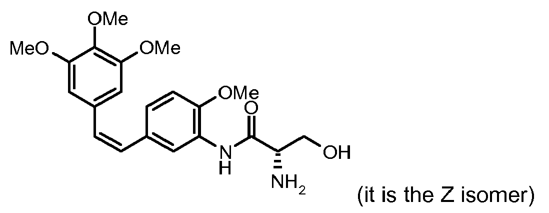 It is an antivascular agent (or VDA, Vascular Disrupting Agent). It has the chemical name: (Z)-N-[2-methoxy-5-[2-(3,4,5-trimethoxyphenyl)vinyl]phenyl]-L-serinamide. This compound, which is described in EP 731085 B1, may be prepared according to the method described in WO 03/084919. Ombrabulin may be administered in base form (cf. above formula) or in the form of a salt of a pharmaceutically acceptable acid, for example in the form of the hydrochloride, represented below: 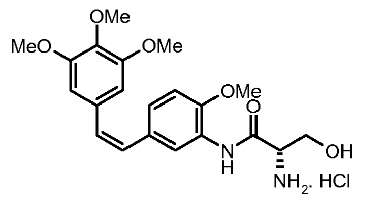 Once administered, ombrabulin releases *in vivo* the active metabolite (Z)-1-(3-amino-4-methoxyphenyl)-2-(3,4,5-trimethoxyphenyl)ethene, which has the formula: 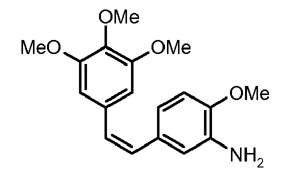 It is therefore also possible to substitute, for ombrabulin, another combretastatin of formula: 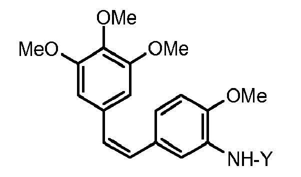 in base form or in the form of a salt of a pharmaceutically acceptable acid, in which Y represents an amino acid, which releases *in vivo* this metabolite.

Bevacizumab (Avastin® - Genentech/Roche) is a humanized monoclonal antibody that recognizes and blocks vascular endothelial growth factor (VEGF). VEGF is a chemical signal that stimulates the growth of new blood vessels (angiogenesis). This compound and its preparation are disclosed in US 6,054,297.

The combination comprises an effective quantity of ombrabulin and an effective quantity of bevacizumab.

Ombrabulin may be administered by infusion at a dose comprised between 8 and 50mg/m², for example selected from the following doses: 8; 11.5; 15.5; 20; 25; 35; 50 mg/m² or intermediate doses.
Bevacizumab may be administered by infusion at a dose between 5 and 10 mg/kg, for example selected from the following doses: 5 or 10 mg/kg.

The cycle of administration of both anti-tumoral agents is repeated with an interval of three weeks.

The invention also concerns the use of ombrabulin and bevacizumab for the preparation of an anti-tumoral combination that is disclosed above in this document.

The invention also concerns the above disclosed anti-tumoral pharmaceutical combination comprising ombrabulin and bevacizumab; ombrabulin being in the form of a free base or of an addition salt with a pharmaceutical acceptable acid, or in the form of a hydrate or of a solvate, for its use as a medicament in the treatment of advanced solid tumors.

### Definitions

- pharmaceutically acceptable acid: organic or inorganic acid having a low toxicity (see "Pharmaceutical salts" J.Pharm.Sci. 1977, 66, 1-19);
- effective amount: amount of a pharmaceutical compound that produces an effect on the treated tumor.
- advanced solid tumors: locally advanced or metastatic solid tumors ie tumors which are not operable anymore.

The combination is administered repeatedly in a course of several cycles according to a protocol that depends on the nature and on the stage of the cancer to be treated, as well as the status of patient to be treated (age, weight, previous treatment(s), etc.).

Examples of cycles and doses are given in the study below.

An open-label, non-randomized, dose escalation, safety and pharmacokinetics phase I study of ombrabulin in combination with bevacizumab administered by intravenous infusion every 3 weeks in patients with advanced solid tumors is disclosed below.

### STUDY OBJECTIVE(S)

Primary objectives:
- To determine the maximum administered dose (MAD) and the maximum tolerated dose (MTD) of ombrabulin in combination with best tolerated dose of bevacizumab based on the incidence of related Dose Limiting Toxicities (DLTs).

Secondary objectives:
- To assess the overall safety profile of the combination
- To characterize the pharmacokinetic (PK) profile of both ombrabulin and bevacizumab when given in combination
- To evaluate preliminary evidence of anti-tumor activity of the combination: RECIST 1.1 for solid tumors or both the international workshop response criteria and the revised response criteria for non Hodgkin lymphoma (NHL) and Hodgkin lymphoma patients (HL)
- To assess the pharmacodynamic effect using DCE-US, measuring circulating biomarkers
- To evaluate potential predictive biomarkers on tumor samples

### STUDY DESIGN

This is an open-label, non-randomized, dose escalation, safety, pharmacodynamic and pharmacokinetic evaluation of ombrabulin given in combination with bevacizumab administered 24 hours after the end of ombrabulin infusion.

The combination will be administered every 3 weeks to patients with advanced solid tumors according to the following schedule:
Day 1 T0: ombrabulin administered as a 30 minutes i.v. infusion
Day 2 T0: bevacizumab administered as a 30-90 minutes i.v. (decreasing with cycles) infusion 24 hours after the end of ombrabulin infusion.

Cohorts of 3 patients will be treated at each dose level. The decision to escalate to the following dose level will be based on the number of patients experiencing drug-related DLTs during cycle 1 among all treated patients. At each decision point regarding dose-escalation, all cycle 1 DLTs related to the treatment from all patients treated at all tested dose levels will be taken into account to estimate the dose-toxicity relationship.

A Bayesian dose-escalation design will be used to determine the dose level to be given to the next cohort of patients and finally estimate the MTD of the combination (Bailey, 2009; Neuenschwander, 2008). A minimum of one cycle duration (i.e. 3 weeks) will be mandatory between the last patient who has been treated at dose level n, and the first patient of next cohort who will be treated at the next dose level.

The first 3-patient cohort (level la) will receive ombrabulin 8 mg/m² on D1 combined with bevacizumab 5 mg/kg on D2.
If the dose-escalation criterion is met, then a second cohort of 3 patients (level lb) will receive ombrabulin 8 mg/m² and bevacizumab 10 mg/kg.
A third 3-patient cohort (level IIa) will receive ombrabulin 11.5 mg/m² combined with bevacizumab 5 mg/kg.
If the dose-escalation criterion is met, then a fourth cohort of 3 patients (level llb) will receive ombrabulin 11.5 mg/m² and bevacizumab 10 mg/kg.
If the dose-escalation criterion is met after this dose level, then following cohorts of patients will receive escalating doses of ombrabulin (15.5, 20, 25, 35 and 50 mg/m²) on Day 1 of each cycle with a fixed dose of 10 mg/kg bevacizumab on D2 assuming that safety parameters are acceptable at the end of cycle 1 for each dose.
According to the model dose-escalation guidelines, the dose of bevacizumab could also be decreased to 5 mg/kg.
The dose escalation is stopped as soon as 2 cohorts of 3 patients (i.e. 6 patients) have been treated at the same dose level and the dose escalation model still recommends current dose level as the MTD when the two cohorts are completed (model's recommendation is to treat a new cohort at the same dose level). This will define the maximum tolerated dose for the combination.
When the MTD is reached, an expanded cohort of 10 patients will be treated at this MTD level in order to assess safety and anti-tumor activity of the combination.
Although the dose escalation process is primarily guided by evaluation of DLTs related to the treatment during cycle 1, toxicities observed in subsequent cycles should also be considered for the dose escalation decision (i.e. smaller increases in dose, expansion of a given dose level, evaluation of optional intermediate doses) and for the dose selection. However, these additional data will not be taken into account in the Bayesian model which will only use cycle 1 data. Thus, these additional data will be considered as supportive descriptive information. It could be decided, if necessary, to test other intermediate dose levels than those mentioned by the current protocol (i.e. bevacizumab between 5-10 mg/kg). The following dose levels are planned:

| **Ombrabulin** **(mg/m²)** | **Bevacizumab** **5 mg/kg** | **Bevacizumab** **10 mg/kg** | **Bevacizumab Other dose between 5-10 mg/kg** |
|---|---|---|---|
| **8** | X | X | O |
| **11.5** | X | X | O |
| **15.5** | O | X | O |
| **20** | O | X | O |
| **25** | O | X | O |
| **35** | O | X | O |
| **50** | O | X | O |

| | | | |
|---|---|---|---|
| *X: dose levels initally planned* *O : optional intermediate* dose *levels* | | | |

### STUDY POPULATION

### Main inclusion criteria

- Histologically or cytologically proven solid malignant tumor with the exception of squamous non small cell lung cancer (NSCLC).
- Advanced neoplastic disease (i.e. metastatic or locally unresectable advanced disease); NHL (non Hodgkin lymphoma) and HL (Hodgkin lymphoma) in progression after standard therapy and for which intensive therapy is not indicated could be included.

### Main exclusion criteria

- Age < 18 years old
- ECOG performance status > 1
- Life expectancy less than 12 weeks

### Related to the methodology

- Concurrent treatment with any other anticancer therapy
- Female subjects not post-menopausal, not surgically sterile or not using effective contraception.

### Related to the disease

- Pericardial effusion requiring intervention (drainage)
- History of brain metastasis, ongoing unstable symptomatic spinal cord compression or carcinomatous meningitis or new evidence of brain metastasis on screening MRI scan
- Diagnosis of squamous NSLCL or with mixed cell type with predominant squamocellular histology or with cavitation
- Hormone sensitive prostate cancer
- Extended abdominal /pelvic radiotherapy and mediastinal radiation that encompasses any field of the heart
- Major surgery within 28 days of study enrollment or surgical wound not fully healed before study enrollment
- High cumulative dose of anthracycline (i.e. doxorubicin > 400 mg/m² or epirubicin > 750 mg/m²)

### Related to the study drugs

- Urine protein-creatinin ratio (UPCR)>1 (urinalysis on morning spot urine) or proteinuria> 500mg/24h
- Serum creatinin ≥ 1.5 x ULN and/or creatinin clearance calculated according to Cockroft-Gault formula < 60ml/min
- Inadequate hematology function including: neutrophils < 1.5 x 10⁹/L; platelets < 100 x 10⁹/L; hemoglobin < 9.0 g/dL; in case of bone marrow involvement in lymphoma patients, no haematological criteria are required
- Liver function: Total bilirubin not within normal limit and ALT/AST ≥ 2.5 ULN. Alkaline phosphatase up to grade 2 would be accepted only if related to the presence of bone metastases (bone specific isoenzyme AP to be evaluated)
- Any of the following within 6 months prior to study enrollment: peptic ulcer disease, erosive oesophagitis or gastritis, infectious or inflammatory bowel disease and diverticulitis
- Patients receiving anticoagulation
- Congenital bleeding diathesis or acquired coagulopathy
- Recent hemoptysis (occurring within the past 3 months)
- Hypersensitivity to the active substance or to any of the excipients (hydrochloric acid, trehalose dihydrate, sodium phosphate, polysorbate 20)
- Discontinuation of previous anti-VEGF agents (single or combined) for toxicity reason
- Previous treatment with ombrabulin or any vascular disrupting agents

### Related to Cardiovascular effects

All patients must see a cardiologist within four weeks prior to the first infusion to validate their eligibility on the absence of the following cardiovascular exclusion criteria:
- Any of the following within 6 months prior to study enrollment: myocardial infarction, coronary/peripheral artery bypass graft, documented angina pectoris, congestive heart failure, arrhythmia especially severe conduction disorders (second or third-degree atrio-ventricular block), cerebrovascular accident or transient ischemic attack, arterial or venous thromboembolism requiring anticoagulants and pulmonary embolism
- Patient with a LVEF < 50% (or inferior to the lower normal limit of the center) by echocardiography
- ST segment depression or elevation ≥ 1 mm in at least 2 contiguous leads or presence of infarction Q-waves on 12-lead ECG: Cardiac troponin (cTnl) at levels that exceed the normal ranges values defined by the laboratory
- Patient with grade 2 retinopathy at ocular fundoscopy
- Patient with uncontrolled hypertension or untreated hypertension defined as systolic BP > 160 mmHg or diastolic BP > 90 mmHg on two repeated measurements at 30-minute interval. Of note, the diagnosis of hypertension should be substantiated by repeated readings performed at least on two different days before considering hypertension as possible diagnosis.

### PRODUCTS

Route(s) of administration:

Ombrabulin and bevacizumab will be administered by intravenous infusion:
- Over 30 min for ombrabulin;
- As per SpC of bevacizumab, the required I.V. administration is: first infusion over 90 min, second over 60 min and later infusions over 30 min. However for pharmacokinetics purpose, the two first infusion will last 90 min, the third one 60 minutes and the following ones 30 minutes.

### PROTOCOL

### PHARMACOKINETICS:

Blood samples for pharmacokinetic analyses of the two compounds will be obtained from all patients at Cycles1 & 2, and before infusion of bevacizumab at Cycle 3 for pharmacokinetic analyses of bevacizumab only.

### Ombrabulin:

### Cycle1 and Cycle 2, Day 1

A series of 1 mL blood samples will be collected (i.e. a total of 11 mL of blood per cycle):
- immediately prior to the end of infusion
- post infusion: 5, 10, 25 and 45 minutes; 1, 2, 4, 6, 8 hours

### Cycle1 and Cycle 2, Day 2;

- one blood sample 24 hours post ombrabulin infusion and before bevacizumab infusion) *Bevacizumab:*

### Cycle1 and Cycle 2, Day 2

A series of 5 mL blood samples will be collected (i.e. a total of 35 mL of blood at cycle 1 and cycle 2):
- prior to the start of infusion
- immediately prior to the end of bevacizumab infusion, 2 and 6 hours after the end of infusion

### Cycle1 and Cycle 2, Days 4, 8 and 15 ;

- one blood sample 48 hours, 6 and 13 days post bevacizumab infusion Cycle 3
- one blood sample 5 ml before bevacizumab infusion.

### PHARMACODYNAMY:

### DCE-US:

- Cycle 1: before infusion of ombrabulin, 24 hours after the end of ombrabulin infusion (D 2, before bevacizumab infusion) and Day 8
- Cycle 2: 24 hours after the end of ombrabulin infusion (D2, before bevacizumab infusion), Day 8 and Day 21
- Even cycles: Day 21

### STATISTICAL CONSIDERATIONS

The primary objective of the trial is to find the MTD. The MTD is defined as the dose with maximum confidence that the risk of DLT associated to the dose lies in an interval ranging from 20% to 35% (i.e. targeted interval).

A Bayesian dose-escalation design will be used to determine the MTD of the combination (Bailey, 2009; Neuenschwander, 2008).
A Bayesian logistic 3-parameter model will be used to guide the dose escalation. Patients will be treated sequentially at a given dose level by cohorts of 3 patients. After each cohort of 3 patients, the Bayesian model will provide the posterior distribution of risk to have DLT for each dose level, based on the cumulative current data observed. The posterior distribution will used to identify the dose(s) with the highest probability to be in the targeted interval (i.e. 20 to 35%). If the candidate doses are higher than the current dose level, the next dose level can be tested.
Statistical and graphical guidelines will be provided regarding the dose to be given to the next cohort and the estimation of risk of DLT at each dose level. The dose given to the next cohort will thus be chosen according to these guidelines and also taking into account the full clinical picture of patients previously treated. In particular, information regarding toxicities observed after cycle 1 will not be taken into account in the Bayesian model but could be used as supportive descriptive information to help decision-making on dose escalation.
After each cohort, five options are possible:
- Treat 3 patients at next planned higher dose level
- Treat 3 patients at next higher optional dose level
- Treat 3 additional patients at the same dose level
- Treat 3 additional patients at a lower dose level
- Stop the dose escalation and consider the current dose as the MTD.
The dose escalation will be stopped when 2 consecutive cohorts of 3 patients have been treated at a same dose level and the dose escalation model recommends current/lower dose level as the MTD when the two cohorts are completed. This will define the maximum tolerated dose for the combination
The sample size will depend on the toxicity of the combination and would be in a range of approximately 40 to 70 patients in case of completion of the full planed dose levels (bevacizumab 5 mg/kg).
The safety population will be the all treated population defined as the subset of all registered patients exposed to ombrabulin or bevacizumab, regardless of the amount of treatment administered.
Activity/efficacy population is defined as all registered patients who have received at least two cycles of ombrabulin and bevacizumab, and provide a baseline and at least one post-baseline assessment for the efficacy variable of interest. Patients with an early progression as per RECIST 1.1 will also be included in this set.

The primary safety analysis will assess DLT separately for cycle 1 and other cycles. The secondary safety analysis will summarize treatment emergent adverse events (TEAEs) and post-TEAE by actual dose level in the all treated patient population. Adverse events will be summarized with respect to frequency, incidence, intensity/severity (as graded by the NCI CTCAE, version 4.0), relationship to the study treatment, and cumulative nature. All adverse events will be coded using the MedDRA system. Serious TEAE, post-TEAE and deaths will be tabulated.
Pharmacokinetics parameters will be summarized with descriptive statistics. Statistical modelling will be used to assess dose proportionality of Cmax and AUC and dose effect. Anti-tumor activity as assessed by the best overall response using RECIST 1.1 or both the international workshop response criteria and the revised response criteria will be descriptively presented by actual dose level in the activity/efficacy population.

### DURATION OF STUDY PERIOD

### (per Patient)

The duration of the study for each patient will include:
- an up to 28-day screening phase
- 21-day study treatment cycles,
- an end of treatment visit ( 30 days after the last treatment infusion)
- follow-up visits or patient contact every 30 days until further anti cancer therapy, death or progression for patient who discontinued treatment for safety reason
- an end of study visit

The cut-off date (COD) for the global analysis of the study and for the issuing clinical report is defined as the date when the last treated patient will have his fourth cycle course completed (30-day post infusion = cut-off date) or theoretical date if the last patient received less than 4 cycles, at a maximum. After this COD, patients will receive the treatments until disease progression, occurrence of an adverse event (or death) leading to treatment discontinuation, which ever come the first; they will only be followed for IP administration, further therapy, SAEs, drug related AEs and AEs leading to study treatment discontinuation.

## Claims

1. An anti-tumoral combination comprising ombrabulin and bevacizumab, ombrabulin being in the form of a free base or of an addition salt with a pharmaceutical acceptable acid, or in the form of a hydrate or of a solvate.

2. Combination according to claim 1 where this antitumoral combination is well tolerated, does not exacerbate the toxicity of each of the antitumoral agents and which allows the treatment of advanced solid tumors either by stabilizing or by inducing a partial or a complete regression of the tumor.

3. Combination according to claim 1 or 2 where ombrabulin is in the form of the hydrochloride salt.

4. Combination according to any one of claims 1 to 3 comprising an effective quantity of ombrabulin and an effective quantity of bevacizumab.

5. Combination according to claim 4 where ombrabulin is administered by perfusion at a dose comprised between 8 and 50mg/m².

6. Combination according to claim 5 where the dose is selected from the following doses: 8; 11.5; 15.5; 20; 25; 35; 50 mg/m².

7. Combination according to claim 4 where bevacizumab is administered by perfusion at a dose comprised between 5 and 10 mg/kg.

8. Combination according to claim 7 where the dose is selected from the following doses: 5 or 10 mg/kg.

9. Combination according to any one of claims 1 to 8, where the cycle of administration of the both antitumoral agents is repeated with an interval between two administrations of three weeks.

10. Use of ombrabulin and bevacizumab for the preparation of an antitumoral combination as claimed in claims 1 to 9.

11. An anti-tumoral pharmaceutical combination comprising ombrabulin and bevacizumab, ombrabulin being in the form of a free base or of an addition salt with a pharmaceutical acceptable acid, or in the form of a hydrate or of a solvate, for its use as a medicament in the treatment of advanced solid tumors.
